# FASCICULE DE BREVET EUROPEEN

(11) **EP 0 679 100 B1**
(45) Date de publication et mention de la délivrance du brevet: **03.07.1996**
(21) Numéro de dépôt: 94928995.3
(22) Date de dépôt: 14.10.1994
(51) Int. Cl.: A61M 1/16, G01F 25/00

(54) **CIRCUIT DE LIQUIDE DE DIALYSE**
DIALYSEFLüSSIGKEITSKREISLAUF
DIALYSIS LIQUID CIRCUIT

(30) Priorité: 15.10.1993 IT TO930757
(43) Date de publication de la demande: 02.11.1995
(73) Titulaire: HOSPAL AG, CH-4008 Basel (CH)
(72) Inventeur: STELLATI, Monica, I-40141 Bologna (IT)
(74) Mandataire: Lejeune, Daniel
(86) Numéro de dépôt international: IB9400317
(87) Numéro de publication internationale: WO9510311

(56) Documents cités:
- EP-A- 0 243 284

## Description

La présente invention concerne un circuit de liquide de dialyse pour une machine de dialyse.

Plus particulièrement, l'invention concerne un circuit de liquide de dialyse comprenant :
- une canalisation d'alimentation en liquide de dialyse frais connectable à une entrée d'un premier compartiment d'un dialyseur, cette canalisation étant munie de premiers moyens de mesure d'une caractéristique du liquide de dialyse,
- une canalisation d'évacuation de liquide usé connectable à une sortie du premier compartiment d'un dialyseur, cette canalisation étant munie de seconds moyens de mesure d'une caractéristique du liquide de dialyse,
- des moyens de dérivation pour relier sur commande la canalisation d'alimentation à la canalisation d'évacuation et isoler le dialyseur, et
- des moyens pour étalonner les moyens de mesure.

Un tel circuit est décrit dans le brevet européen 0 243 284.

Les canalisations d'alimentation et d'évacuation sont reliées par les moyens de dérivation notamment lors de phases d'étalonnage des moyens de mesure programmées à intervalles réguliers pendant le fonctionnement de la machine. Les demandes de brevet européens 0 418 171 et 0 579 559 mis à la disposition du public après la date de priorité de la présente invention. Décrivent des procédés d'étalonnage adaptés à ce type de circuit où les moyens de mesure sont des débitmètres.

Ce circuit présente un inconvénient pour l'étalonnage de certains types de moyens de mesure (débitmètres à engrenages par exemple) lié au fait que le liquide qui circule au contact des seconds moyens de mesure durant le fonctionnement normal de la machine de dialyse (liquide usé) n'est pas le même que celui qui circule au contact de ces moyens de mesure lors des phases d'étalonnage (liquide de dialyse frais). En particulier, les viscosités de ces liquides sont différentes, leurs températures étant généralement différentes et le liquide usé contenant des substances organiques que le liquide de dialyse frais ne contient pas, par définition.

Un but de l'invention est de réaliser un circuit de liquide de dialyse du type mentionné ci-dessus qui permette d'étalonner les moyens de mesure dans des conditions de mesure aussi proches que possible de celles du fonctionnement de traitement, où le liquide de dialyse circule dans le dialyseur.

Pour atteindre ce but, on prévoit, selon l'invention, un réservoir tampon pour liquide usé disposé sur la canalisation d'évacuation en amont des seconds moyens de mesure pour permettre la circulation de liquide usé au contact des seconds moyens de mesure lorsque les canalisations d'alimentation et d'évacuation sont reliées par les moyens de dérivation au moins pendant le temps nécessaire à l'étalonnage des moyens de mesure.

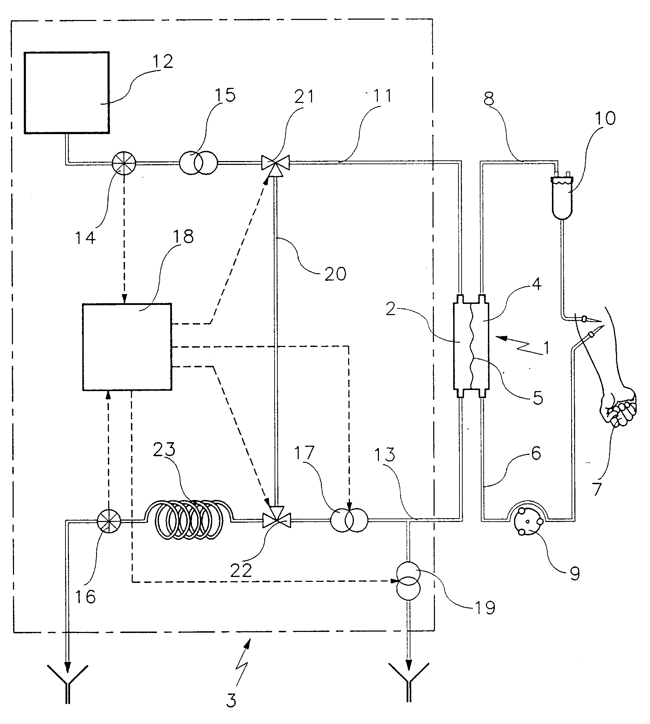

D'autres caractéristiques et avantages de l'invention apparaîtront à la lecture de la description qui va suivre, faite en relation au dessin annexé sur lequel l'unique figure représente de façon schématique un circuit de liquide de dialyse selon l'invention.

Sur la figure, on a représenté un rein artificiel en fonctionnement comprenant un échangeur à membrane semi-perméable 1 (hémodialyseur) ayant un premier compartiment 2 connecté au circuit de liquide de dialyse d'une machine de dialyse 3 et un second compartiment 4, séparé du premier compartiment 2 par une membrane semi-perméable 5, connecté à un patient par un circuit sang.

Le circuit sang comprend une canalisation de prélèvement 6 ayant une extrémité munie d'une canule enfoncée dans une artère d'un patient 7 et son autre extrémité connectée à une entrée du second compartiment 4 du dialyseur 1, et une canalisation de restitution 8 ayant une extrémité connectée à une sortie du second compartiment 4 du dialyseur 1 et son autre extrémité munie d'une canule enfoncée dans une veine du patient 7. Sur la canalisation de prélèvement 6 est disposée une pompe de circulation 9. La canalisation de restitution 8 comprend un piège à bulles 10.

Le circuit de liquide de dialyse comprend une canalisation d'alimentation 11 en liquide de dialyse frais ayant une extrémité reliée à une source de liquide de dialyse 12, tel qu'un générateur de liquide de dialyse, et son autre extrémité connectée à une entrée du premier compartiment 2 du dialyseur 1, et une canalisation d'évacuation 13 de liquide usé ayant une extrémité connectée à une sortie du second compartiment 2 du dialyseur 1 et son autre extrémité reliée à l'égout.

Sur la canalisation d'alimentation 11 sont disposés des premiers moyens de mesure 14 d'une caractéristique du liquide de dialyse frais, tels qu'un débitmètre, et une première pompe de circulation 15. Sur la canalisation d'évacuation 13, sont disposés des seconds moyens de mesure 16 d'une caractéristique du liquide usé, tels qu'un débitmètre, et une seconde pompe de circulation 17. Le premier débitmètre 14 est situé en amont de la première pompe 15 et le second débitmètre 16 est situé en aval de la seconde pompe 17. La seconde pompe de circulation 17 est pilotée par une unité de calcul et de commande 18 en fonction des débits mesurés par les débitmètres 14 et 16, de façon que ces débits soient égaux. Grâce à cette disposition, il est possible d'extraire du circuit de liquide de dialyse, au moyen d'une pompe d'extraction 19 connectée à la canalisation d'évacuation 13 en amont de la deuxième pompe 17, une quantité précise de liquide usé correspondant exactement à la quantité de liquide plasmatique qu'il est prescrit de faire perdre au patient. La mise en service de la pompe d'extraction 19 par l'unité de commande 18 provoque l'ultrafiltration de liquide plasmatique au travers de la membrane 5, du second compartiment 4 vers le premier compartiment 2.

Le circuit de liquide de dialyse comprend en outre des moyens de dérivation permettant de relier la canalisation d'alimentation 11 à la canalisation d'évacuation 13 de façon à isoler le dialyseur 1 et à disposer les deux débitmètres 14 et 16 directement en série lois de phases d'étalonnage des débitmètres commandées à intervalles réguliers. Les moyens de dérivation comprennent une canalisation de dérivation 20 connectée aux canalisations d'alimentation 11 et d'évacuation 13 par l'intermédiaire de deux vannes trois voies 21, 22 respectivement. La vanne 21 est disposée sur la canalisation d'alimentation 11 en aval de la première pompe de circulation 15 et la vanne 22 est disposée sur la canalisation d'évacuation 13 en aval de la seconde pompe de circulation 17.

Conformément à l'invention, le circuit de liquide de dialyse comprend un réservoir tampon 23 disposé sur la canalisation d'évacuation 13 entre la vanne 22 et le débitmètre 16. Ce réservoir tampon 23, qui peut être constitué par une portion de canalisation, a un volume suffisant pour que, compte tenu du débit de la pompe 15 lors de l'étalonnage (identique ou proche du débit de traitement) et de la durée d'une phase d'étalonnage des débitmètres 14, 16, il ne circule dans le second débitmètre 16, lors de l'étalonnage, que du liquide usé. De la sorte, les conditions dans lesquelles les mesures sont effectuées par le second débitmètre 16 lors de l'étalonnage sont les mêmes que lors du fonctionnement de traitement, le débitmètre étant parcouru dans les deux cas par du liquide de dialyse usé. Grâce à cette disposition, il est possible de calculer des coefficients d'étalonnage précis.

Le dispositif qui vient d'être décrit fonctionne de la manière suivante.

Avant le début d'une séance de traitement, le circuit de liquide de dialyse, l'hémodialyseur 1 et le circuit extracorporel de sang 6, 8, 10 sont rincés et remplis de solution saline. Après quoi, le circuit extracorporel de sang est connecté au patient 7, d'abord par la canalisation de prélèvement 6, puis, lorsque sous l'effet de la rotation de la pompe de circulation 9, le circuit est presque totalement rempli de sang et purgé de la solution saline qui le remplissait, par la canalisation de restitution 8.

Le traitement commence lorsque le liquide de dialyse, préparé par le générateur de liquide de dialyse 12, est mis en circulation dans le circuit de liquide de dialyse par les pompes 15, 17. Les vannes trois voies 21 et 22 sont alors disposées de façon à autoriser la circulation de liquide dans les canalisations d'alimentation 11 et d'évacuation 13, et à interdire la communication entre chacune de ces canalisations et la canalisation de dérivation 20. Le débit du liquide de dialyse frais est mesuré par le débitmètre 14 et il est comparé, par l'unité de calcul et de commande 18, au débit de liquide usé qui est mesuré par le débitmètre 16. L'unité de commande 18 pilote la seconde pompe de circulation 17 de façon que ces débits soient égaux. L'unité de commande 18 pilote également la pompe d'extraction 19 en fonction d'un débit d'ultrafiltration de consigne.

A intervalles réguliers, les débitmètres sont étalonnés. Pour ce faire, les vannes 21 et 22 sont disposées de façon à autoriser la circulation de liquide de dialyse frais dans la canalisation de dérivation 20. De la sorte, les débitmètres 14 et 16 sont reliés directement en série et les mesures qu'ils effectuent dans cette configuration correspondent au même débit de liquide. Comme le volume du réservoir tampon 23 est choisi pour que, au débit d'étalonnage choisi (en général égal au débit de traitement), et pendant le temps nécessaire à l'étalonnage, il ne s'écoule, au travers du second débitmètre 16, que du liquide usé, la mesure qui est effectuée par ce débitmètre, pendant la phase d'étalonnage, est effectuée dans les conditions de traitement. Un coefficient d'étalonnage exact peut ainsi être calculé par l'unité de calcul et de commande 18 à partir des mesures effectuées par les deux débitmètres 14, 16 pendant la durée de chaque phase d'étalonnage.

## Revendications

1. Circuit de liquide de dialyse comprenant :
- une canalisation d'alimentation (11) en liquide de dialyse frais connectable à une entrée d'un premier compartiment (2) d'un dialyseur (1), cette canalisation étant munie de premiers moyens (14) de mesure d'une caractéristique du liquide de dialyse,
- une canalisation d'évacuation (13) de liquide usé, connectable à une sortie du premier compartiment (2) d'un dialyseur (1), cette canalisation étant munie de seconds moyens de mesure (16) d'une caractéristique du liquide de dialyse,
- des moyens de dérivation (20, 21, 22) pour relier sur commande la canalisation d'alimentation (11) à la canalisation d'évacuation (13) de façon à disposer les moyens de mesure (14, 16) directement en série, et
- des moyens (18) pour étalonner les moyens de mesure (14, 16), caractérisé en ce qu'il comporte un réservoir tampon (23) pour liquide usé disposé sur la canalisation d'évacuation (13) en amont des seconds moyens de mesure (16) pour permettre la circulation de liquide usé au contact des seconds moyens de mesure (16) lorsque les canalisations d'alimentation (11) et d'évacuation (13) sont reliées par les moyens de dérivation au moins pendant le temps nécessaire à l'étalonnage des moyens de mesure (14, 16).

2. Circuit selon la revendication 1, caractérisé en ce que le réservoir tampon est constitué par une portion (23) de la canalisation d'évacuation.

3. Circuit selon une des revendications 1 et 2, caractérisé en ce que les premiers et seconds moyens de mesure sont des débitmètres (14, 16).

## Claims

1. Dialysis liquid circuit, comprising:
- a feed line (11) for fresh dialysis liquid, which can be connected to an inlet of the first compartment (2) of a dialyser (1), this line being provided with first means (14) for measuring a characteristic of the dialysis liquid,
- a line (13) for discharging used liquid, which can be connected to an outlet of the first compartment (2) of a dialyser (1), this line being provided with second means (16) for measuring a characteristic of the dialysis liquid,
- bypass means (20, 21, 22) for connecting the feed line (11) to the discharge line (13) on demand, so as to arrange the measurement means (14, 16) directly in series, and
- means (18) for calibrating the measurement means (14, 16), characterized in that it includes a buffer reservoir (23) for used liquid, arranged on the discharge line (13) upstream of the second measurement means (16), in order to allow used liquid to flow in contact with the second measurement means (16) when the feed (11) and discharge (13) lines are connected by the bypass means, at least for the time needed to calibrate the measurement means (14, 16).

2. Circuit according to Claim 1, characterized in that the buffer reservoir consists of a portion (23) of the discharge line.

3. Circuit according to one of Claims 1 and 2, characterized in that the first and second measurement means are flowrate meters (14, 16).

## Patentansprüche

1. Dialysierflüssigkeitskreislauf, der folgendes umfaßt:
- eine Zufuhrleitung (11) für frische Dialysierflüssigkeit, die mit einem Eingang eines ersten Faches (2) eines Dialysators (1) verbunden werden kann, wobei diese Leitung mit ersten Mitteln (14) zur Messung einer Eigenschaft der Dialysierflüssigkeit ausgestattet ist,
- eine Ablaufleitung (13) für verbrauchte Flüssigkeit, die mit einem Ausgang des ersten Faches (2) eines Dialysators (1) verbunden werden kann, wobei diese Leitung mit zweiten Mitteln (16) zur Messung einer Eigenschaft der Dialysierflüssigkeit ausgestattet ist,
- Umleitungsmittel (20, 21, 22) zur Verbindung, auf Befehl, der Zufuhrleitung (11) mit der Ablaufleitung (13), so daß die Meßmittel (14, 16) direkt in Reihe geschaltet werden, und
- Mittel (18) zum Eichen der Meßmittel (14, 16), dadurch gekennzeichnet, daß er einen Puffertank (23) für verbrauchte Flüssigkeit umfaßt, der stromaufwärts der zweiten Meßmittel (16) an der Ablaufleitung (13) angeordnet ist, um das Fließen von verbrauchter Flüssigkeit in Kontakt mit den zweiten Meßmitteln (16) zu gestatten, wenn die Zufuhrleitung (11) und Ablaufleitung (13) durch die Umleitungsmittel miteinander verbunden sind, und zwar zumindest während der für das Eichen der Meßmittel (14, 16) erforderlichen Zeit.

2. Kreislauf nach Anspruch 1, dadurch gekennzeichnet, daß der Puffertank aus einem Teil (23) der Ablaufleitung besteht.

3. Kreislauf nach Anspruch 1 oder 2, dadurch gekennzeichnet, daß es sich bei den ersten und zweiten Meßmitteln um Durchflußmesser (14, 16) handelt.
